# EUROPEAN PATENT APPLICATION

(11) **EP 1 243 654 A1**
(43) Date of publication of application: **25.09.2002**
(21) Application number: 02003238.9
(22) Date of filing: 20.02.2002
(51) Int. Cl.: C12N 15/29, C07K 14/415, C12N 15/82, C12N 5/10

(54) **Precursor polypeptides of phytosulfokine derived from Arabidopsis thaliana and cDNAs encoding said polypeptides**

(30) Priority: 27.02.2001 JP 2001052946
(71) Applicant: NAGOYA UNIVERSITY, Nagoya-shi, Aichi Pref. 464 (JP)
(72) Inventor: Sakagami, Yoji, Nagoya City, Aichi Pref. (JP); Matsubayashi, Yoshikatsu, Nagoya City, Aichi Pref. (JP); Yang, Heping, Nagoya City, Aichi Pref. (JP)
(74) Representative: Wibbelmann, Jobst, Dr., Dipl.-Chem.

(57) **Abstract**

For the purpose to promote proliferation of a plant cell, a novel gene is provided by this invention. This invention provides a precursor polypeptide of phytosulfokine that enhances proliferation of a plant cell derived from *Arabidopsis Thaliana*, named *AtPSK2* polypeptide. This invention further provides *AtPSK2* gene that encodes the *AtPSK2* polypeptide. Moreover, this invention provides another precursor polypeptide of phytosulfokine that enhances proliferation of a plant cell derived from *Arabidopsis Thaliana*, named *AtPSKL* polypeptide. This invention further provides *AtPSKL* gene that encodes the *AtPSKL* polypeptide. Proliferation of a plant cell can be achieved by incorporation of the gene according to this invention.

## Description

### BACKGROUND OF THE INVENTION

### 1. Field of the invention

This invention relates to precursor polypeptide of phytosulfokine derived from *Arabidopsis thaliana* and a gene encoding the precursor polypeptide. The phytosulfokine is a peptide, which enhances proliferation of a plant cell. Moreover, this invention relates to a method to promote proliferation of a plant cell by incorporation of the gene into a plant.

### 2. Description of Related Art

Caused by the development of the technique of plant gene engineering, enthusiastic progression was achieved on production of a transgenic plant wherein various exogenous genes are incorporated. Such technique is available for various plant species and plays an important role in development of industries. For example, such technique enables production of novel plant species with improved productivity of their secondary metabolites.

For the production of a transgenic plant, it is necessary to cultivate small number of transformed cells, wherein a certain exogenous gene is incorporated to regenerate a plant body. However, in the case of a plant cell wherein a certain exogenous gene is incorporated, proliferation of such cell is very slow. Therefore, regeneration of a plantlet to produce a transgenic plant may be difficult. A Plant cell secretes unknown growth factors into extracellular medium to promote cell proliferation. However, when the plant cells exist in lower density clusters than necessary, proliferation of the plant cell becomes difficult. Because it takes too many times for the growth factor to reach sufficient concentration, or degradation rate of the growth factor proceeds that of secretion. Moreover, in many plant species, cell culture itself is difficult or the rate of cell proliferation is very slow. Therefore, the development of a technique available for promotion of plant cell proliferation has been desired.

### SUMMARY OF THE INVENTION

To achieve such purpose, the inventors noticed and investigated on phytosulfokine (PSK), which is a peptidyl plant growth factor. The PSK is one of the plant growth factors contained in so-called "conditioned medium: CM", a medium once used for cell culture. It is known that the PSK is secreted extracellular medium and functions like autocrine. In general, a known hormone or various nutritional elements are added to the culture medium. However, in some plant species, the cultivation itself is difficult or the proliferation is difficult. In addition, the cultivation of the cell becomes difficult when the cell density is very low. In such cases, phytosulfokine (PSK) is effective to activate proliferation of a plant cell. It is known that tyrosine residue of PSK is sulfated by post-translational modification. Moreover, two types of PSK, namely PSK-α and PSK-β, are known to exist. It is also known that PSK-α and PSK-β are peptides consisting of the structures as described below, wherein their tyrosine residues are sulfated by post-translational modification. The PSK-β is an enzymatic degradation product of PSK-α and the cell proliferation activity observed in PSK-β is less than one-tenth, when compared to that of PSK-α.
PSK-α: Tyr(SO₃H)-Ile-Tyr(SO₃H)-Thr-Gln
PSK-β: Tyr(SO₃H)-Ile-Tyr(SO₃H)-Thr

Incidentally, it is known that the PSK-α and PSK-β are bio-synthesized as their precursors, then the PSKs are sulfated and processed during transition via trans-Golgi network. Concerning the cDNA sequence encoding *Oryza sativa* phytisulfokine (OsPSK), which is one of such precursors, its sequence has already determined by the inventors, using the technique of cDNA cloning. A patent application was filed on the cDNA base sequence thus obtained and on the amino acid sequence encoded by the cDNA, and the patent application was allowed in Japan as Japanese Patent No. 3038381.

As described above, a phytosulfokine precursor polypeptide and a gene encoding the polypeptide were known on rice. As widely known, rice is a monocotyledonous plant with a significant industrial importance. Therefore, obtaining the phytosulfokine precursor polypeptide derived from rice and the gene encoding the polypeptide deserves a significant value. However, in a dicotyledonous plant, the phytosulfokine precursor polypeptide and the gene encoding the polypeptide were not obtained and the sequences defining them were not determined so far. Until now, PSK was detected from monocotyledonous plants such as rice and asparagus, as well as from dicotyledonous plants such as *Arabidopsis thaliana,* zinnia and carrot. Considering it, the gene encoding phytosulfokine precursor polypeptide is assumed to exist universally in the plant kingdom to play an important role in proliferation of a plant cell.

The plant kingdom has a diversity and there are monocotyledonous and dicotyledonous plants, which exist in the kingdom. In addition, there is a great industrial demand on the phytosulfokine precursor polypeptide.
Therefore, it is very valuable to obtain the phytosulfokine precursor polypeptide and the gene encoding the polypeptide derived from a dicotyledonous plant. As well, *Arabidopsis thaliana* is adopted as a representative model plant in the field of plant genetics. Because *Arabidopsis thaliana* has some advantageous characters, such as, the size of the plant body is small, the time scale of one generation is short, the size of the genome is small. Therefore, among many dicotyledonous plants, the inventors have chosen *Arabidopsis thaliana* as the target plant to be analyzed. The sequences of the phytosulfokine precursor polypeptide and the gene encoding the polypeptide derived from *Arabidopsis thaliana* were obtained and determined, which is the object of this invention.

The first aspect of this invention is the *AtPSK2* polypeptide, which is a precursor polypeptide of phytosulfokine derived from *Arabidopsis thaliana.* The polypeptide is characterized by being following (a) or (b):
(a) a polypeptide consisting of an amino acid sequence defined as amino acid numbers from 1 to 87 in SEQ ID NO: 1 in the sequence list,
(b) a polypeptide consisting of an amino acid sequence having more than 70% of sequence homology with said polypeptide (a), said polypeptide (b) being processed and sulfated on the tyrosine residue of said phytosulfokine in a plant cell to secrete said phytosulfokine with its tyrosine residue sulfated, thereby capable of promoting proliferation of said plant cell.

The second aspect of this invention is a gene encoding the *AtPSK2* polypeptide, a precursor polypeptide of phytosulfokine derived from *Arabidopsis thaliana.* The gene is characterized by being following (1) to (3),
(1) A gene encoding the *AtPSK2* polypeptide.
(2) A gene encoding the *AtPSK2* polypeptide, the gene consisting of a base sequence defined as base numbers from 40 to 303 in SEQ ID NO: 2 in the sequence list.
(3) A gene encoding a precursor polypeptide of phytosulfokine derived from *Arabidopsis thaliana*, the gene being following (c), (d) or (e):
   (c) a gene consisting of a base sequence defined as base numbers from 1 to 545 in SEQ ID NO: 2 in the sequence list,
   (d) a gene consisting of a base sequence that hybridizes with the base sequence (c) under a stringent condition,
   (e) a gene consisting of a base sequence having more than 70% of sequence homology with said gene (c), said gene (e) encoding a polypeptide being processed and sulfated on the tyrosine residue of said phytosulfokine in a plant cell to secrete said phytosulfokine with its tyrosine residue sulfated, thereby capable of promoting proliferation of said plant cell.

The third aspect of this invention is the *AtPSKL* polypeptide, a precursor polypeptide of phytosulfokine derived from *Arabidopsis thaliana.* The polypeptide is characterized by being following (f) or (g):
(f) a polypeptide consisting of an amino acid sequence defined as amino acid numbers from 1 to 79 in SEQ ID NO: 3 in the sequence list,
(g) a polypeptide consisting of an amino acid sequence having more than 70% of sequence homology with said polypeptide (f), said polypeptide (g) being processed and sulfated on the tyrosine residue of said phytosulfokine in a plant cell to secrete said phytosulfokine with its tyrosine residue sulfated, thereby capable of promoting proliferation of said plant cell.

The forth aspect of this invention is a gene encoding the *AtPSKL* polypeptide, a precursor polypeptide of phytosulfokine derived from *Arabidopsis thaliana.* The gene is characterized by being following (4) to (6),
(4) A gene encoding the *AtPSKL* polypeptide.
(5) A gene encoding the *AtPSKL* polypeptide, the gene consisting of a base sequence defined as base numbers from 43 to 282 in SEQ ID NO: 4 in the sequence list.
(6) A gene encoding a precursor polypeptide of phytosulfokine derived from *Arabidopsis thaliana,* the gene being following (h), (i) or (j):
   (h) a gene consisting of a base sequence defined as base numbers from 1 to 479 in SEQ ID NO: 4 in the sequence list,
   (i) a gene consisting of a base sequence that hybridizes with the base sequence (h) under a stringent condition,
   (j) a gene consist of a base sequence having more than 70% of sequence homology with said gene (h), said gene (j) encoding a polypeptide being processed and sulfated on the tyrosine residue of said phytosulfokine in a plant cell to secrete said phytosulfokine with its tyrosine residue sulfated, thereby capable of promoting proliferation of said plant cell.

The history of obtaining the precursor polypeptide of phytosulfokine derived from *Arabidopsis thaliana* is as follows. The inventors induced callus from roots of *Arabidopsis thaliana*, then produced a cDNA library from the callus. PCR was performed with suitably designed probes, using the cDNA as a template. As a result, some bands were revealed to be amplified, sequence analysis of the bands was performed. Then the sequence encoding PSK was detected in two clones. The cDNA library was screened using the sequence as the probes, two clones designated as *AtPSK2* and *AtPSKL* were obtained.

The sequences of these clones were determined. The result revealed that *AtPSK2* was a gene consisting of total 545 bases and the gene encoded precursor polypeptide of phytosulfokine consisting of 87 amino acids. Moreover, *AtPSKL* was the gene consisting of total 479 bases and the gene encoded precursor polypeptide of phytosulfokine consisting of 79 amino acids. The *Arabidopsis* cultured cells were transformed by the *AtPSK2* gene or the *AtPSKL* gene. As a result, it was confirmed that both genes could secret the PSK. Moreover, when the cultured *Arabidopsis* cells were transformed by the *AtPSK2* gene or the *AtPSKL* gene, it was confirmed that cell proliferation was enhanced in the transformed *Arabidopsis* cultured cells.

These and other objects and advantages of the invention will become more apparent upon a reading of the detailed description and drawings.

### Brief description of the drawings

Figure 1 is a figure showing the base sequence of the *AtPSK2* gene and the deduced amino acid sequence.

Figure 2 is a figure showing the base sequence of the *AtPSKL* gene and the deduced amino acid sequence.

Figure 3 is a photograph of DNA blot analysis indicating the copy number of the *AtPSK2* gene.

Figure 4 is a photograph of DNA blot analysis indicating the copy number of the *AtPSKL* gene.

Figure 5 is a figure showing the alignment of the *AtPSK2* and the rice phytosulfokine precursor polypeptide (OsPSK).

Figure 6 is a figure showing the alignment of the *AtPSKL* and the rice phytosulfokine precursor polypeptide (OsPSK).

Figure 7 is a figure showing the alignment of the *AtPSK2* and the *AtPSKL.*

Figure 8 is a photograph of the DNA blot analysis showing the introduced *AtPSK2* gene in the cells transformed with the *AtPSK2* gene.

Figure 9 is a photograph of the DNA blot analysis showing the introduced *AtPSKL* gene in the cells transformed with the *AtPSKL* gene.

Figure 10 is a photograph of the blotting analysis, detecting the amount of the *AtPSK2* transcripts in the sense transgenic cells and in the antisense transgenic cells of *AtPSK2.*

Figure 11 is a photograph of the blotting analysis, detecting the amount of the *AtPSKL* transcripts in the sense transgenic cells and in the antisense transgenic cells of *AtPSKL.*

Figure 12 is a photograph comparing growth of the sense transgenic cells and the antisense transgenic cells harboring *AtPSK2.*

Figure 13 is a photograph comparing growth of the sense transgenic cells and the antisense transgenic cells harboring *AtPSKL.*

Figure 14 is a photograph showing the time course of the expression of the *AtPSK2* gene.

Figure 15 is a photograph showing the time course of the expression of the *AtPSKL* gene.

Figure 16 is a photograph showing the tissue specificity of the expression of the *AtPSK2* gene.

Figure 17 is a photograph showing the tissue specificity of the expression of the *AtPSKL* gene.

### DETAILED DESCRIPTIONS OF THE INVENTION

This invention relates to the *AtPSK2* gene, a gene encoding precursor polypeptide of phytosulfokine derived from *Arabidopsis thaliana.* Here, precursor polypeptide of phytosulfokine means a polypeptide, the polypeptide including phytosulfokine in its sequence, being processed and sulfated on the tyrosine residue of the phytosulfokine in a plant cell to secrete the phytosulfokine with its tyrosine residue sulfated, thereby capable of promoting proliferation of said plant cell. The gene of this invention is specified by a gene consisting of a base sequence defined as base numbers from 1 to 545 shown in SEQ ID NO: 2 in the sequence list. In the base sequence shown in SEQ ID NO: 2 in the sequence list, base numbers from 40 to 303 corresponds to the open reading frame, which encodes the *AtPSK2* polypeptide described below.

According to the technique of gene recombination, artificial modification can be achieved at a specific site of basic DNA, without alteration or with improvement of basic characteristic of said DNA. Concerning a gene having native sequence provided according to this invention or modified sequence different from said native sequence, it is also possible to perform artificial modification such as insertion, deletion or substitution to obtain gene of equivalent or improved characteristic compared with said native gene. Moreover, a gene with such mutation is also included in the range of this invention.

That is, the gene, consisting of a base sequence that hybridizes with the base sequence shown in SEQ ID NO: 2 in the sequence list under a stringent condition, means a gene in which 20 or less, preferably ten or less, and more preferably five or less bases of the sequence is deleted, substituted or added to the base sequence shown in SEQ ID NO: 2 in the sequence list. Moreover, such gene exhibits homology 70% or more, preferably 80% or more and still preferably 90% or more with the base sequence shown in SEQ ID NO: 2 in the sequence list. In addition, such gene hybridizes with the base sequence shown in the SEQ ID NO: 2 in the sequence list under a stringent condition. Such gene is also within the range of this invention so far as it encodes a polypeptide exhibiting the characteristic as a precursor polypeptide of phytosulfokine, being processed and sulfated on the tyrosine residue of the phytosulfokine in a plant cell to secrete the phytosulfokine with its tyrosine residue sulfated, thereby capable of promoting proliferation of said plant cell.

Furthermore, this invention relates to the *AtPSK2* polypeptide, which is the precursor polypeptide of phytosulfokine derived from *Arabidopsis thaliana.* The *AtPSK2* polypeptide is specified by an amino acid sequence of amino acid numbers from 1 to 87 shown in SEQ ID NO: 1 in the sequence list. This polypeptide is encoded by open reading frame portion of the base sequence indicated by SEQ ID NO: 2 in the sequence list.

The polypeptide consisting of an amino acid sequence in which a part of said polypeptide referred to as amino acid sequence shown in SEQ ID NO: 1 is deleted, substituted or added with another amino acid sequence means a polypeptide in which 20 or less, preferably ten or less, and more preferably five or less amino acids of the sequence is deleted, substituted or added to the amino acid sequence shown in SEQ ID NO: 1 in the sequence list. Moreover, such polypeptide exhibits homology 70% or more, preferably 80% or more and still preferably 90% or more with the amino acid sequence shown in SEQ ID NO: 1 in the sequence list. Such polypeptide is also within the range of this invention so far as it exhibits characteristic as a precursor polypeptide of phytosulfokine, being processed and sulfated on the tyrosine residue of the phytosulfokine in a plant cell to secrete the phytosulfokine with its tyrosine residue sulfated, thereby capable of promoting proliferation of said plant cell.

Furthermore, this invention relates to *AtPSKL* gene, which is also a gene encoding precursor polypeptide of phytosulfokine derived from *Arabidopsis thaliana.* The gene of this invention is defined by a base sequence consisting of base numbers from 1 to 479 shown in SEQ ID NO: 4 in the sequence list. In the base sequence shown in SEQ ID NO: 4 in the sequence list, base numbers from 43 to 282 corresponds to the open reading frame which encodes *AtPSKL* polypeptide described below.

According to the technique of gene recombination, artificial modification can be achieved at a specific site of basic DNA, without alteration or with improvement of basic characteristic of said DNA. Concerning a gene having native sequence provided according to this invention or modified sequence different from said native sequence, it is also possible to perform artificial modification such as insertion, deletion or substitution to obtain gene of equivalent or improved characteristic compared with said native gene. Moreover, a gene with such mutation is also included in the range of this invention.

That is, the gene, consisting of a base sequence that hybridizes with said base sequence shown in SEQ ID NO: 4 in the sequence list under stringent condition, means a gene in which 20 or less, preferably ten or less, and more preferably five or less bases of the sequence is deleted, substituted or added to the base sequence shown in SEQ ID NO: 4 in the sequence list. Moreover, such gene exhibits homology 70% or more, preferably 80% or more and still preferably 90% or more with the base sequence shown in SEQ ID NO: 4 in the sequence list. In addition, such gene hybridizes with the base sequence shown in the SEQ ID NO: 4 in the sequence list under a stringent condition. Such gene is also within the range of this invention so far as it encodes a polypeptide exhibiting the characteristic as a precursor polypeptide of phytosulfokine, being processed and sulfated on the tyrosine residue of the phytosulfokine in a plant cell to secrete the phytosulfokine with its tyrosine residue sulfated, thereby capable of promoting proliferation of said plant cell.

Furthermore, this invention relates to *AtPSKL* polypeptide, which is the precursor polypeptide of phytosulfokine derived from *Arabidopsis thaliana.* The *AtPSK2* polypeptide is specified by an amino acid sequence of amino acid numbers from 1 to 79 shown in SEQ ID NO: 3 in the sequence list. This polypeptide is encoded by open reading frame portion of the base sequence indicated by SEQ ID NO: 4 in the sequence list.

The polypeptide consisting of an amino acid sequence in which a part of said polypeptide referred to as amino acid sequence shown in SEQ ID NO: 3 is deleted, substituted or added with another amino acid sequence means a polypeptide in which 20 or less, preferably ten or less, and more preferably five or less amino acids of the sequence is deleted, substituted or added to the amino acid sequence shown in SEQ ID NO: 3 in the sequence list. Moreover, such polypeptide exhibits homology 70% or more, preferably 80% or more and still preferably 90% or more with the amino acid sequence shown in SEQ ID NO: 3 in the sequence list. Such polypeptide is also within the range of this invention so far as it exhibits characteristic as a precursor polypeptide of phytosulfokine, being processed and sulfated on the tyrosine residue of the phytosulfokine in a plant cell to secrete the phytosulfokine with its tyrosine residue sulfated, thereby capable of promoting proliferation of said plant cell.

A method to transform a plant by incorporating the *AtPSK2* gene or the *AtPSKL* gene into a plant, and a transgenic plant cell produced by incorporation of the *AtPSK2* gene or the *AtPSKL* gene are also within the range of this invention. The *AtPSK2* gene and the *AtPSKL* gene according to this invention are genes that encode precursor polypeptides of phytosulfokine, as described above. Therefore, as shown in the following embodiment, it was confirmed that transformation of a plant cell by incorporation of the *AtPSK2* gene or the *AtPSKL* gene resulted in secretion of phytosulfokine into the medium. Moreover, it was also confirmed that cell proliferation was enhanced in a plant cell transformed by the *AtPSK2* gene or the *AtPSKL* gene.

Therefore, proliferation of a plant cell can be enhanced by incorporation of the *AtPSK2* gene or the *AtPSKL* gene. The example of plants, preferred as a target plant to which the *AtPSK2* gene or the *AtPSKL* gene according to this invention is incorporated, may include monocotyledonous plants, such as, lily, rice, maize, asparagus and wheat, as well as dicotyledonous plants, such as *Arabidopsis thaliana*, tobacco, carrot, soybean, tomato, potato and zinnia. In principle, any plant can be adopted as a target plant to which the *AtPSK2* gene or the *AtPSKL* gene according to this invention is incorporated, to enhance proliferation of a plant cell derived from the plant.

A conventional method known in this art can be utilized as a method to produce a transformant. A vector available in this invention may include a plasmid vector, such as pMAT037 utilized in the following embodiment, as well as pIG122, pBI101, pBI121, pBI221, pAct-nos/Hmz, pTA7001 and pTA7002. However, the range of the vector is not to be limited to them, other plasmids conventionally utilized in this art can be also adopted. Such vector can be incorporated into, for example, an *Agrobacterium* strain, then a callus or a plantlet can be transfected by the *Agrobacterium* strain to produce a transgenic plant. Furthermore, a seed from such transgenic plant can be obtained. The method to incorporate the plant gene according to this invention is not to be limited to the *Agrobacterium-mediated* method and other methods, such as particle gun method and electroporation method, can be also adopted for incorporation of the plant gene according to this invention.

Moreover, a plant body derived from the transformed plant cell thus produced to enhance cell proliferation, as well as a method to produce a transformed plant cell, is also within the range of this invention. Examples described above and embodiments described below are preferred embodiments of this invention and are not intended to exhibit limitation or range of this invention.

The experiments are performed as described below.

### (Plant Materials)

Seeds of *Arabidopsis thaliana* ecotype Columbia (Collection number Col-0) were purchased from Lehle Seeds (Round Rock, TX, USA), sown on vermiculite and grown in a greenhouse at 25°C under continuous illumination from daylight fluorescent tubes providing 150 mmol m⁻²s⁻¹ (FL40SBRN, Toshiba, Tokyo, Japan). Seedlings 28 days after germination were used as the source of RNA for northern blot analysis.

### (Establishment of Arabidopsis Cell Suspensions)

*Arabidopsis* seeds (Collection number Col-0) were surface-sterilized for 5 min in 70% EtOH, washed three times with sterile distilled water, transferred to 5% NaOCl for 10 min, rinsed five times with sterile distilled water, and placed on 150 × 25 mm Petri dishes containing Murashige and Skoog (MS) medium (Murashige and Skoog, 1962) to germinate. Plants were grown at 25°C in a 16-hr light/8-hr dark cycle. The same growth-room conditions were used for tissue culture procedures. Roots were collected from 28-day-old seedlings and cultured on B5 medium (Gamborg et al., 1968) supplemented with 0.5 mg/l 2,4-D and 0.05 mg/l KIN. Calli obtained from the explants were subcultured on the same medium at regular intervals of 2 weeks.

### (Genomic DNA Blot Analysis)

Genomic DNA was extracted from the calli using the CATB method (Murry and Thompson, 1980). Aliquots of 10 µg DNA were digested with restriction endonucleases, separated on 0.8% (w/v) agarose gels, and transferred to Biodyne nylon filters (Pall, New York, NY, USA) in 20 × SSC. Hybridization was executed in a solution of 5 × SSC, 0.5% SDS, 5 × Denhardt's solution, and 500 µg/ml Salmon sperm DNA at 50°C and 65°C using the ³²P-labeled probe prepared with a Random Primed DNA Labeling Kit (Takara, Shiga, Japan). Washing after hybridization was performed with 2 × SSC at 25°C for 15 min 3 times and then 2 × SSC containing 0.1% SDS at 50°C and 65°C for 15 min 3 times.

### (Screening of Libraries)

From the ten-days cultured callus, poly⁺ (A) mRNA was purified by oligo (dT) column and cDNA library was constructed using λgt10 system (Takara, Tokyo, JAPAN). To amplify DNA fragments encoding PSK-α precursor(s), DNA (100 ng) from the cDNA library was used as a template for amplification of partial cDNA with a λgt 10 forward primer and a mixture of 15-mer degenerated primers [5'-T(C)TGG(A)GTG(A)TAG(A)ATG(A)TA-3'] complementary to the deduced oligonucleotides from which the amino acid sequence of PSK-α can be deduced. PCR was run for 30 cycles each of which consisted of denaturation at 95°C for 1 min, primer annealing at 35°C for 1 min, and polymerization at 72°C for 1 min in a Robocycler (Stratagene, La Jolla, CA, USA). The PCR products were ligated into the pCR 2.1 vector (Invitrogen, Carlsbad, CA, USA), and the specific cDNA fragment was then used to screen the cDNA library and the genomic library by plaque hybridization. Hybridization was performed as described above.

### (DNA Sequence Analysis)

The inserts of positive phages were subcloned into pBS SK- plasmids (Stratagene). *Escherichia coli* strain JM109 was used as the host for the plasmids. Deletion clones were generated for DNA sequencing with a Kilo Sequencing Kit (Takara) according to the protocol recommended by the manufacturer. The plasmid DNA templates were amplified with a BigDye Terminator Cycle Sequencing Kit (Applied Biosystems, Foster, CA, USA), and sequenced with an ABI PRISM 310 Genetic Analyzer (Applied Biosystems) in accordance with the manufacturer's protocols.

### (Construction Of Chimeric Genes)

A 22-mer primer (5'-TCTTCTGGGAATAGATGTAATC-3') based on the nucleic acid sequence of the *AtPSK2* cDNA and A 24-mer primer (5'-CTGAGAATAAATGTAATCGGTGTG-3') based on the nucleic acid sequence of the *AtPSKL* cDNA were synthesized and used to obtain the mutated cDNAs that were designed to produce unnatural [Ser⁴]PSK-α instead of PSK-α. Mutations were introduced with a LA PCR *In Vitro* Mutagenesis Kit (Takara) and confirmed by sequencing. The pMAT037 (Matsuoka and Nakamura, 1991) was employed as a binary vector for *Arabidopsis* root transformation. Original or mutated cDNAs were digested with *Sac*I and *Hin*dIII or *Kpn*I and inserted in-frame into the same sites of the vectors to construct chimeric genes harboring the cDNA in sense or antisense orientations, respectively. Expression of the chimeric genes was driven by the promoter of the CaMV 35S transcript.

### (Transformation of Arabidopsis Roots)

Constructs were transformed into *Agrobacterium tumefaciens* (C58C1Rif^{R}) by triparental mating (Van Haute et al., 1983), and *Agrobacterium*-mediated transformation of roots of *Arabidopsis* Columbia ecotype was essentially as described (Valvekens et al., 1988). Transgenic microcalli were generated by incubating co-cultivated root explants for 3 weeks on the callus induction medium (B5 + 0.5 mg/l 2,4-D and 0.05 mg/l KIN) supplemented with 50 mg/l Kan sulphate (Life Technologies, Grand Island, NY, USA) and 750 mg/l vancomycin hydrochloride (Wako, Osaka, Japan). The individual regenerating microcalli were transferred to fresh medium to ensure that all transformants were due to independent T-DNA insertions. To detect the introduced genes, genomic DNA was isolated from transgenic calli, digested with *Bam*HI, and allowed to hybridize with the labeled cDNA as described above. *Arabidopsis* cells transformed with the binary vector alone served as controls.

### (Purification of PSKs from CM)

CM was prepared from 14-day cultured calli by filtration (Advantec no. 2) and stored at -20°C until use. Eighty ml aliquots of each CM were buffered by adding Tris to a final concentration of 20 mM, adjusted to pH 8.0 with 6.0 N HCl, and then applied to a DEAE Sephadex A-25 column (1.7 × 8 cm, Pharmacia, Piscataway, NJ, USA) which was first equilibrated with 20 mM Tris-HCl buffer at pH 8.0. The column was washed with 50 ml of equilibration buffer and eluted successively with 50 ml of buffer containing 0, 400, 800, then 1200 mM KCl at a flow rate of 60 ml/h. TFA was added to a final concentration of 0.1% into the last two fractions containing PSKs, and then the samples were applied to a Sep-Pak Vac column (12cc, C18, Millipore, Tokyo, Japan) after equilibration with 0.1% TFA. The column was washed with 30 ml of the same buffer at a flow rate of 60 ml/h and eluted with 30 ml of 30% acetonitrile containing 0.1% TFA. The fraction containing PSKs was collected and lyophilized for subsequent analyses.

### (LC/MS Analysis)

Mass spectra were obtained using a Fisons VG platform quadruple mass spectrometer with electrospray ionization interfaced to a Jasco PU 980 HPLC system. The fraction containing PSKs was dissolved in 200 µl of water and separated on a reverse-phase HPLC column (Develosil ODS-HG-5, 4.6 × 250 mm, Nomura Chemicals, Seto, Japan) with 10% acetonitrile containing 0.1% TFA at 1.0 ml/min. The HPLC eluate was split 1:9 so that 100 µl/min flowed to the mass spectrometer during the separation. The pseudomolecular ions of PSKs were scanned every 1.9 s with selected ion monitoring at *m*/*z* 831 ([M-H]-of [Ser⁴]PSK-α), *m*/*z* 751 ([M-H-SO₃]⁻ of [Ser⁴]PSK-α), *m*/*z* 845 ([M-H]- of PSK-α), *m*/*z* 765 ([M-H-SO₃]⁻ of PSK-α), *m*/*z* 703 ([M-H]- of [Ser⁴]PSK-β), and *m*/*z* 717 ([M-H]- of PSK-β). The semiquantitative amounts of the PSK-α, PSK-β, [Ser⁴]PSK-α and [Ser⁴]PSK-β in CM were measured based on the peak heights without an internal standard. The fractions containing [Ser⁴]PSK-α or [Ser⁴]PSK-β were collected separately, lyophilized and dissolved in 20 µl of water for sequencing.

### (Amino Acid Sequence Analysis)

Amino acid sequences were determined by Edman degradation with a 490 Procise Protein Sequencing System (Applied Biosystems). Phenylthiohydantoin derivatives of amino acids obtained at each cycle were analyzed by reversed-phase HPLC on an ABI Brownlee C-18 column.

### (Expression Analysis of the AtPSK2 and AtPSKL Genes)

Total RNAs were isolated from *Arabidopsis* roots, leaves, shoots, or culture cells incubated for 1, 3, 7, 10 or 14 days as reported (Chomczynski, 1993). Twenty microgram aliquots were denatured at 65°C for 5 min in 50% formamide, 1 × MOPS (200 mM MOPS, 10 mM EDTA, 50 mM NaOAc, pH 7.0) and 1.5% formaldehyde. The RNAs were then fractionated by electrophoresis on a 1.2% (w/v) agarose gel containing 2.2 M formaldehyde, and subsequently transferred to Biodyne nylon membranes (Pall) in 20 × SSC and then allowed to hybridize with ³²P-labeled cDNA as described above.

The results of experiments according to the above method are described below.

### (Characterization of Arabidopsis Genes for Precursors to PSK)

Poly (A)⁺ RNA was purified from *Arabidopsis* cells cultured for 10-days and used for construction of a cDNA library with the λgt10 vector. Attempts to isolate *Arabidopsis* PSK precursor cDNA using rice PSK precursor (*OsPSK*) cDNA were unsuccessful. In order to obtain a probe for screening of the cDNA library, PCR was carried out using 100 ng of the isolated DNA from the cDNA library. The inventors used degenerated primers complementary to the sequences from which the amino acid sequence of PSK-α can be deduced and the λgt10 forward primer, and a major band of 300-bp was detected. The PCR products were cloned into pBluscript (pBS) SK- vector and sequenced. At least two fragments, designated as *AtPSK2* and *AtPSKL*, were predicted to encode PSK precursors, and they were used as probes to isolate full-length cDNAs corresponding to *AtPSK2* and *AtPSKL*, respectively.

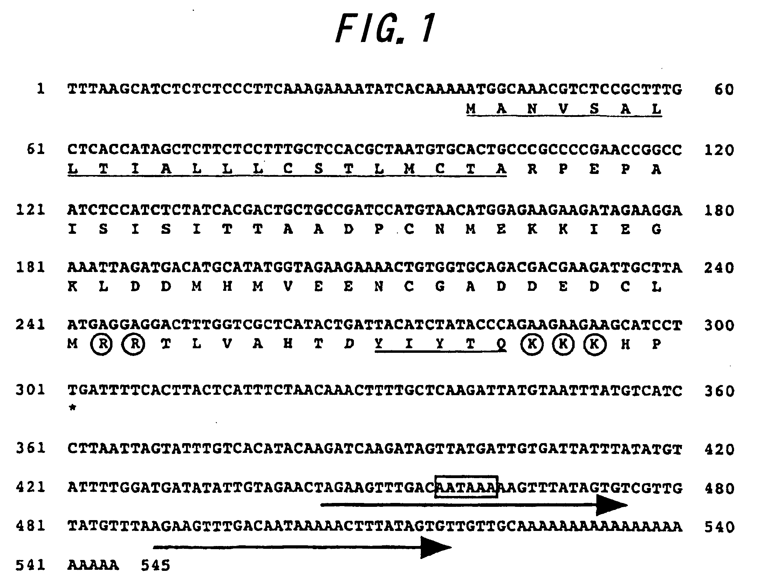

Among 3 × 10⁵ phages screened, 5 clones for *AtPSK2* and 7 clones for *AtPSKL* were obtained. The nucleotide sequences of the largest clones were completely determined on both strands. *AtPSK2* cDNA is 524 bp in length with a polyadenyl tail of 21 adenosine residues (Figure 1). Polyadenylation motifs (AATAAA) were found 19 bp and 62 bp upstream from the polyadenyl tail. Two 35-bp repeats (5'-AGAAGTTTGACAATAAAAAGTTTATAGTGTCGTTG-3' and 5'-AGAAGTTTGACAATAAAAACTTTATAGTGTTGTTG-3') were noted in the 3'-untranslated region (Figure 1). *AtPSK2* cDNA demonstrated an ORF of 261 bp which can code for a precursor to PSK with 87-amino acids (Figure 1) and a predicated molecular mass of 9.6 kDa.

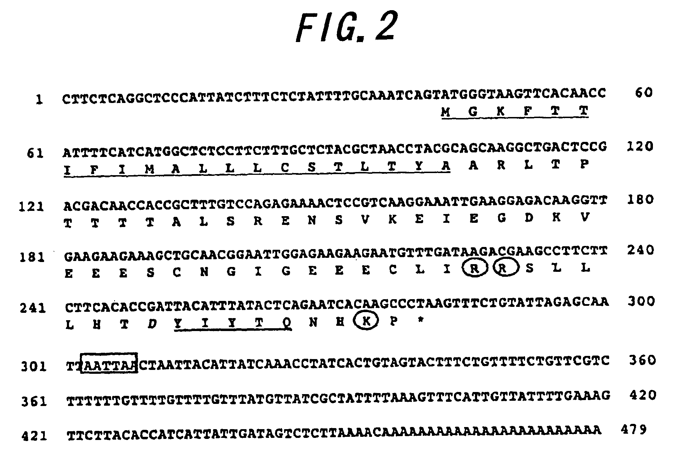

Moreover, the base sequence and deduced amino acid sequence of *AtPSKL* are shown in Figure 2. *AtPSKL* cDNA has no repeats that is shorter than *AtPSK2* cDNA. Accordingly, the precursor deduced from *AtPSKL* cDNA (79-amino acids with a predicated molecular mass of 8.9 kDa) is smaller than that of *AtPSK2.* (Figure 2).

### (Structures of the PSK Precursors Encoded by AtPSK2 and AtPSKL)

As expected, the *AtPSK2* precursor polypeptide (PP-PSK2), a precursor polypeptide encoded by *AtPSK2,* has a potential hydrophobic N-terminal signal sequence (residues 1 through 22), and the prophytosulfokine (P-PSK) is predicted to be 65 amino acids in length (Figure 1). The 5-amino acid PSK-α sequence occurs only once (amino acids 78 through 82) within the PP-PSK2 close to its C-terminus, as shown in Figure 1. There is an aspartic acid residue immediately N-terminal to the first tyrosine of PSK-α in the -1 position (Figure 1), suggesting the tyrosine residues in the PSK-α sequence can be sulfated (Hanai et al., 2000). Interestingly, two Arg (amino acids 69 through 70) residues and three Lys residues (amino acids 83 to 85) exist.

The *AtPSKL* precursor polypeptide (PP-PSKL), a precursor polypeptide encoded by *AtPSKL,* was also found to contain a potential N-terminal signal sequence (amino acids 1 to 21) and one copy of the PSK-α sequence (amino acids 71 to 75) at its C-terminus (Figure 2). The aspartic acid residue is conserved in the -1 position relative to the first tyrosine of PSK-α. Also, two Arg (amino acids 62 through 63) and one Lys (amino acid 78) residues were found to border the PSK-α sequence (Figure 2).

### (Copy Numbers of the Phytosulfokine Precursor Genes)

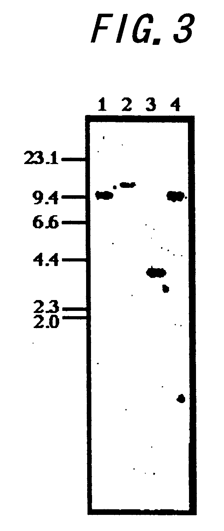

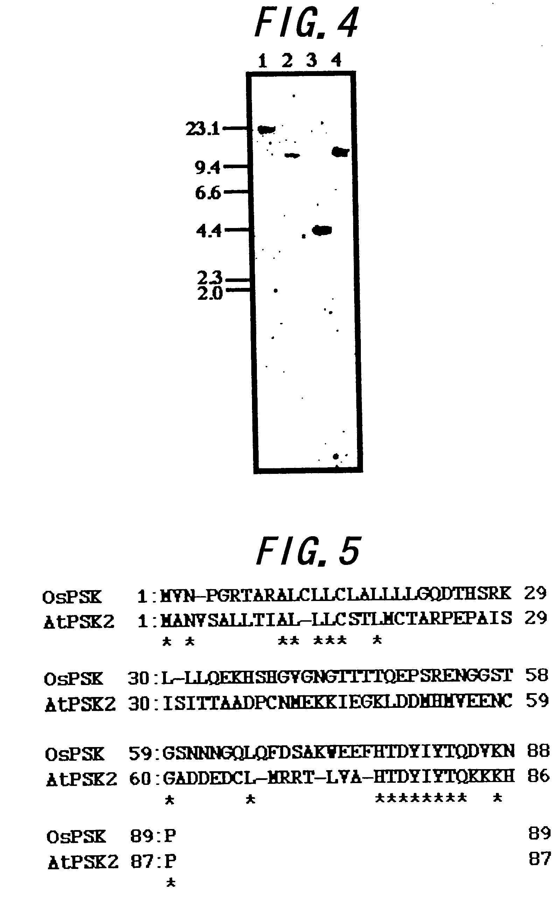

Copy numbers of the *AtPSK2* and *AtPSKL* genes in the *Arabidopsis* genome were investigated by genomic Southern blot analyses. The result in *AtPSK2* and *AtPSKL* are shown in Figures 3 and 4, respectively. In Figures 3 and 4, the results obtained by digestion with BamHI, EcoRI, HindIII and XbaI are shown in lane 1, lane 2, lane 3 and lane 4, respectively. Wild-type DNA was probed with the full-length cDNAs and hybridizations were performed under low- and high- stringency conditions. As results, only one band was detected under either low or highly stringent conditions in the DNA fragments derived from single-digestion of the four different restriction enzymes, indicating that both *AtPSK2* and *AtPSKL* are single-copy genes.

### (Similarity among Arabidopsis and Rice Precursors to PSK)

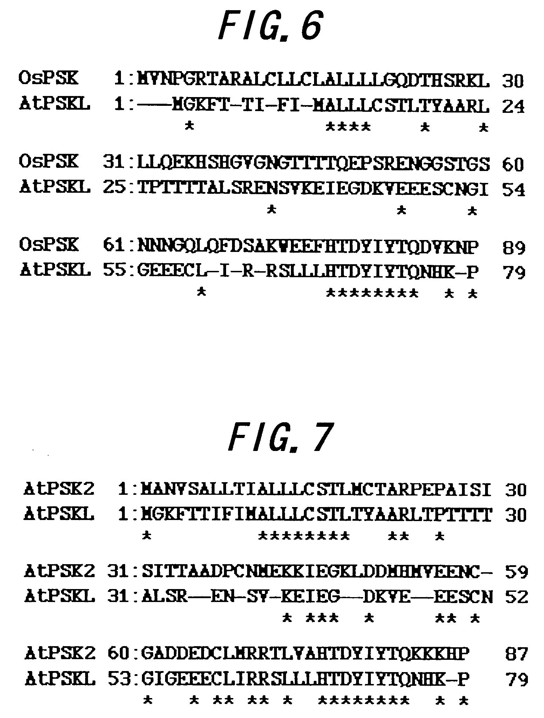

The amino acid sequences of the *Arabidopsis* and rice PSK precursors were aligned using the Clustal W (1.7) Multiple Sequence Alignment provided by the GenomeNet CLUSTALW Server. The result of the alignment between *AtPSK2* and rice preprophytosulfokine (OsPSK) is shown in Figure 5, the alignment between *AtPSKL* and OsPSK is shown in Figure 6 and the alignment between *AtPSK2* and *AtPSKL* is shown in Figure 7. In Figures 5, 6 and 7, the identical amino acids are indicated by stars (*). Quite unexpectedly, these PP-PSKs do not share significant similarity throughout the whole length, although the PSK-α sequence and 3 amino acid residues immediately N-terminal to it are perfectly conserved. This 8-amino acid region is referred to as the PSK domain. In addition, a very limited similarity was found for N-terminal signal peptides.

### (Expression of PSK Precursor cDNAs in Transgenic Cells)

To examine whether *AtPSK2* and *AtPSKL* indeed code for precursors to PSK, we transformed 28-day-old *Arabidopsis* roots with artificially mutated *AtPSK2* or *AtPSKL* cDNAs in the sense orientation designed to produce [Ser⁴]PSK-α instead of PSK-α, and the wild-type cDNAs in sense and antisense orientations, respectively. The chimeric genes were placed under the control of the constitutive cauliflower mosaic virus (CaMV) 35S promoter incorporated within the binary vector pMAT037 (Matsuoka and Nakamura, 1991). *Arabidopsis* cells transformed with the binary vector alone served as controls. B5 medium (Gamborg et al., 1968) supplemented with 0.5 mg/L 2,4-dichlorophenoxyacetic acid (2,4-D) and 0.05 mg/L kinetin (KIN) was used to induce calli.

Three weeks after infection with *Agrobacteria* containing the chimeric genes, yellowish Kan-resistant calli were formed on the root explants. An average of 1 to 2 regenerating calli per root explant usually formed. Using this method based on kanamycin (Kan) selection, the inventors could reproducibly achieve transformation efficiencies between 57% and 79% (root explants forming Kan-resistant calli per total number of infected root explants). DNA was extracted from randomly chosen Kan-resistant calli and analyzed by DNA gel-blot hybridization.

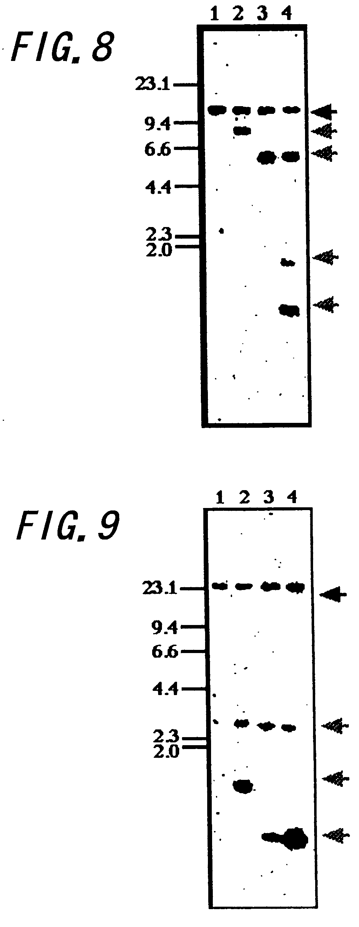

The results on *AtPSK2* and *AtPSKL* is shown in Figures 8 and 9, respectively. In Figure 8, the DNA isolated from the control plants (lane 1), the DNA isolated from the transgenic plants harboring the mutated *AtPSK2* cDNA (lane 2), the DNA isolated from the transgenic plants harboring the sense *AtPSK2* cDNA (lane 3) or the DNA isolated from the transgenic plants harboring the antisense *AtPSK2* cDNA (lane 4) were hybridized with the labeled *AtPSK2* cDNA. In Figure 9, total DNA isolated from the control (lane 1), the transgenic cells harboring the mutated *AtPSKL* cDNA (lane 2), sense (lane 3) or antisense (lane 4) was hybridized with the labeled *AtPSKL* cDNA. In Figures 8 and 9, bands derived from the endogenous (*AtPSK2* or *AtPSKL*) gene and the introduced gene (*AtPSK2* or *AtPSKL*) are indicated by black and stripped arrows, respectively. As results, the introduced genes were detected in all of the transgenic calli (Figure 8 and 9). Around 70% of the transformants had a single locus insertion.

Chimeric genes harboring the mutated cDNAs in-frame were first introduced into *Arabidopsis* root cells. Transgenic cells were obtained at a frequency of 79%, and then one-month-old were transferred to liquid callusinducing medium. Two weeks after the onset of subculture, PSK-α and its analogs in the CM by the transgenic cells were purified and applied to liquid chromatography/mass spectrometry (LC/MS) analysis. Although only PSK-β was detected with transgenic cells containing the pMAT037 vector alone, both PSK-β and [Ser⁴]PSK-β peaks were present in elutes derived from the CM of transgenic cells harboring either the mutated *AtPSK2* or *AtPSKL* cDNAs (Table 1). Furthermore, sequencing of the peptide contained in the corresponding to [Ser⁴]PSK-β fractions confirmed that it indeed was YIYS (data not shown). These results demonstrated that both *AtPSK2* and *AtPSKL* code for precursors to PSK.

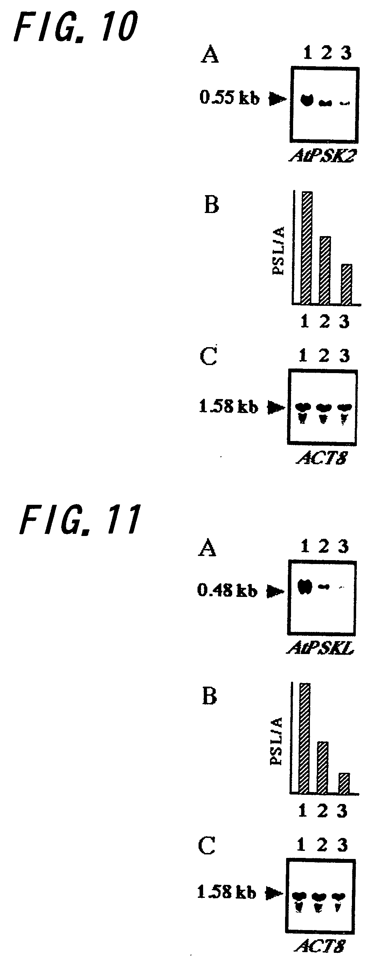

The inventors also introduced wild-type cDNAs in the sense and antisense orientations into *Arabidopsis* cells to examine the effects of supernumerary or suppressed expression of the PSK precursor genes on cell proliferation. Transgenic cells were obtained at frequencies of 73% for the sense chimeric genes and 57% for the antisense constructs. The introduced genes were confirmed by Southern blot analyses (Figures 8 and 9) and the amount of mRNA of the transgenic cells were detected in Figures 10 and 11. The results on *AtPSK2* and *AtPSKL* are shown in Figures 10 and 11, respectively. In Figures 10 and 11, column 1 indicates the result of the sense transgenic cells, column 2 indicates the result of the control cells, column 3 indicates the result of the antisense transgenic cells, respectively. In Figures 10 and 11, A indicates detection of mRNA of *AtPSK2* or *AtPSKL* by blotting, B indicates the quantified results obtained from A, C indicates detection of constitutive expressing actin. Amounts of PP-PSK mRNA were higher in the sense but lower in the antisense transgenic cells than in the controls. That is, the mRNA level increased in the sense transgenic cells and it decreased in the antisense transgenic cells.

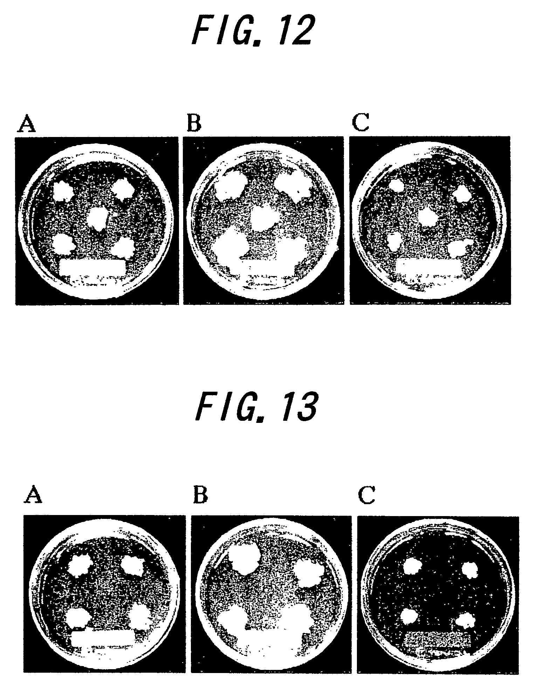

The rate of cell division was investigated on the transgenic cells. The results on *AtPSK2* and *AtPSKL* are shown in Figures 12 and 13, respectively. In Figures 12 and 13, A indicates the result of the sense transgenic cells, B indicates the result of the control cells, C indicates the result of the antisense transgenic cells, respectively. Overexpression of the PSK precursor genes allowed the transgenic cells to divide about two times faster than the controls. In contrast, the transgenic cells in which expression of the PSK precursor genes was suppressed by the antisense cDNA had dramatically decreased mitogenic activity. This was alternated in part by supplementation with PSK-α in the medium (Table 1). These data indicate that both *AtPSK2* and *AtPSKL* genes are involved in plant cell division through the product, PSK-α.

Transgenic cells harboring the sense, antisense, or mutated cDNAs, were quantified for their secretion of PSK analogs into CM. Two weeks after the transgenic cells had been transplanted into fresh media, amounts of the PSKs in CM were measured by LC/MS based on peak heights without an internal standard. Arabidopsis cells containing the binary vector alone served as controls. Consistent with the results of northern blot analyses, PSK accumulated in the CM of the sense transgenic cells at 20 times the control concentration (Table 1). On the other hand, PSK amounts in the CM of the antisense transgenic cells were less than 50% of the mean control level (Table 1). [Ser⁴]PSK-β produced by the transgenic cells with the mutated cDNA reached to more than ten times, compared with the PSK-β derived from the endogenous gene (Table 1).

**Table 1**

| Construct | PSK-β | [Ser⁴]PSK-β |
|---|---|---|
| pMAT037 | 413.5 ± 29.7 | 0 |
| *AtPSK2* mutated | 387.1±40.9 | 3750.5±39.5 |
| sense | 7406.2 ± 57.4 | 0 |
| antisense | 193.6±37.7 | 0 |
| *AtPSKL* mutated | 349.8±35.5 | 4785.2±50.2 |
| sense | 8693.6 ± 79.8 | 0 |
| antisense | 164.9 ± 42.3 | 0 |

### (Expression of the AtPSK2 and AtPSKL Genes)

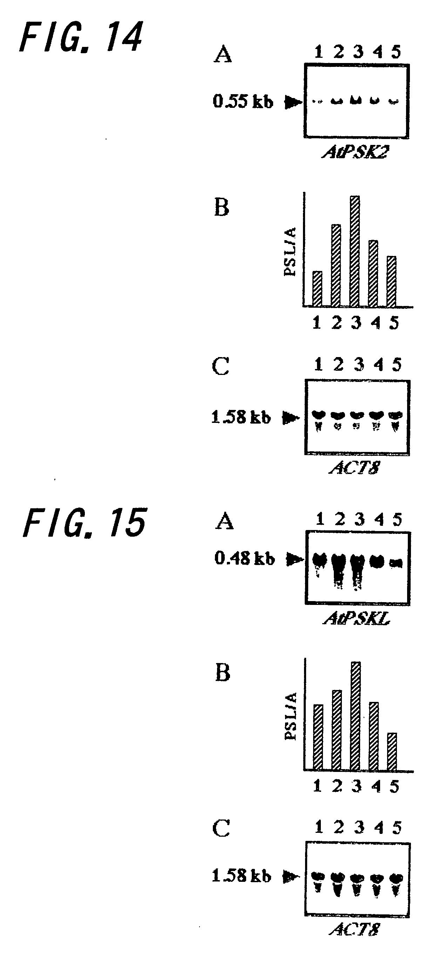

RNA blot analysis with RNA extracted from suspension culture cells revealed the *AtPSK2* gene to be continuously expressed, suggesting provision of a supply of PSK-α, which allows the cells to proliferate rapidly. The time course of RNA content, in accordance to the cultivated days, was analyzed. The results on *AtPSK2* and *AtPSKL* are shown in Figures 14 and 15, respectively. The results are obtained from cells cultured for 1 day (lane 1), 3 days (lane 2), 7 days (lane 3), 10 days (lane 4) and 14 days (lane 5). In Figures 14 and 15, A indicates detection of mRNA of *AtPSK2* or *AtPSKL* by blotting, B indicates the quantified results obtained from A, C indicates detection of constitutive expressing actin. The transcripts increased gradually after cells were transformed to fresh medium and maximal expression values were observed after 7 days to 10 days, followed by a decrease (Figure 14). *AtPSKL* showed an expression pattern similar to that of *AtPSK2,* although expression started earlier, increase of transcripts was faster, and the absolute levels were higher (Figure 15).

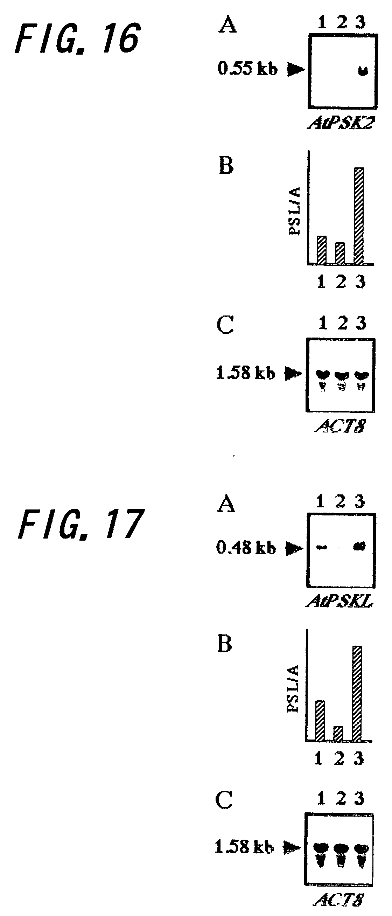

*Arabidopsis* seedlings 28 days after germination were used as source for RNA extraction to perform northern blot analysis. The results on *AtPSK2* and *AtPSKL* are shown in Figures 16 and 17, respectively. In Figures 16 and 17, lane 1 indicates the result obtained from stems, lane 2 indicates the result obtained from leaves, lane 3 indicates the result obtained from roots. In Figures 16 and 17, A indicates detection of mRNA of *AtPSK2* or *AtPSKL* by blotting, B indicates the quantified results obtained from A, C indicates detection of constitutive expressing actin. *AtPSK2* transcripts accumulated in *Arabidopsis* seedlings, whereas the mRNA was most abundant in roots, including apical meristems where cells proliferate vigorously (Figure 16 A). *AtPSKL* was also expressed in the seedlings and most abundantly in roots, but the transcripts in stems including shoot apexes were greater in this case than with *AtPSK2* (Figure 17 A). These findings indicate that the PSK-α molecule is also produced and has physiological functions in intact plants.

This invention provides a precursor polypeptide of phytosulfokine that enhances proliferation of a plant cell derived from *Arabidopsis thaliana,* named *AtPSK2* polypeptide. This invention further provides *AtPSK2* gene that encodes the *AtPSK2* polypeptide. Moreover, this invention provides another precursor polypeptide of phytosulfokine that enhances proliferation of a plant cell derived from *Arabidopsis Thaliana,* named *AtPSKL* polypeptide. This invention further provides *AtPSKL* gene that encodes the *AtPSKL* polypeptide. Proliferation of a plant cell can be achieved by incorporation of the gene according to this invention.

### (REFERENCES)

Brand, U., Fletcher, J.C., Hobe, M., Meyerowitz, E.M., and Simon, R. (2000) Dependence of stem cell fate in *Arabidopsis* on a feedback loop regulated by CLV3 activity. Science 289, 617-619.
Chomczynski P. (1993) A reagent for the single-step simultaneous isolation of RNA, DNA and proteins from cell and tissue samples. BioTechniques 15, 532-536.
Clark, S.E., Williams, R.W., and Meyerowitz, E.M. (1997) The *CLAVATA1* gene encodes a putative receptor kinase that controls shoot and floral meristem size in *Arabidopsis.* Cell 89, 575-585.
Dorner, A.J. and Kaufman, R.J. (1990) Analysis of synthesis, processing, and secretion of proteins expressed in mammalian cells. Methods Enzymol. 185, 577-598.
Fletcher, J.C., Brand, U., Running, M.P., Simon, R., and Meyerowitz, E.M. (1999) Signaling of cell fate decisions by *CLAVATA3* in *Arabidopsis* shoot meristems. Science 283, 1911-1914.
Gamborg, O.L., Miller, R.A., and Ojima, K. (1968) Nutrient requirements of suspension cultures of soybean root cells. Exp. Cell Res. 50, 151-158.
Hanai, H., Matsuno, T., Yamamoto, M., Matsubayashi, Y., Kamada, H., and Sakagami, Y. (2000a) A secreted peptide growth factor, phytosulfokine, acting as a stimulatory factor of carrot somatic embryo formation. Plant Cell Physiol. 41, 27-32.
Hanai, H., Nakayama, D., Yang, H., Matsubayashi, Y., Hirota, Y., and Sakagami, Y. (2000b) Existence of a plant tyrosylprotein sulfotransferase: novel plant enzyme catalyzing tyrosine *O*-sulfation of preprophytosulfokine variants *in vitro.* FEBS Lett. 470, 97-101.
Harris, R.B. (1989) Processing of pro-hormone precursor proteins. Arch. Biochem. Biophys. 275, 315-333.
Huttner, W.B. (1984) Determination and occurrence of tyrosine *O*-Sulfate in protein. Methods Enzymol. 107, 200-223.
Kobayashi, T., Eun, C.-H., Hanai, H., Matsubayashi, Y., Sakagami, Y. and Kamada, H. (1999) Phytosulfokine-α, a peptidyl plant growth factor, stimulates cell division that leads to somatic embryogenesis in carrot. J. Exp. Bot. 50, 1123-1128.
Liu, Y.G., Mitsukawa, N., Lister, N., Dean, C., and Whittier, R.F. (1996) Isolation and mapping of a new set of 129 RFLP markers in *Arabidopsis thaliana* using recombinant inbred lines. Plant J. 10, 733-736.
Matsubayashi, Y. and Sakagami, Y. (1996) Phytosulfokine, sulfated peptides that induced the proliferation of single mesophyll cells of *Asparagus officinalis L.* Proc.Natl. Acad. Sci. USA 93, 7623-7627.
Matsubayashi, Y., Hanai, H., Hara, O. and Sakagami, Y. (1996) Active fragments and analogs of the plant growth factor, Phytosulfokine: structure-activity relationships. Biochem. Biophys. Res. Commun. 225, 209-214.
Matsubayashi, Y., Takagi, L., and Sakagami, Y. (1997). Phytosulfokine-ε, a sulfated pentapeptide, stimulates the proliferation of rice cells by means of specific high- and low-affinity binding sites. Proc.Natl. Acad. Sci. USA 94, 13357-13362.
Matsubayashi, Y., Takagi, L., Omura, N., Morita, A., and Sakagami, Y. (1999) The endogenous sulfated pentapeptide, phytosulfokine-α, stimulates tracheary element differentiation of isolated mesophyll cells of *Zinnia elegans.* Plant Physiol. 120, 1043-1048.
Matsuoka, K. and Nakamura, K. (1991) Prepeptide of a precursor to a plant vacuolar protein required for vacuolar targeting. Proc.Natl. Acad. Sci. USA 88, 834-838.
McGurl, B., Pearce, G., Orozco-Cardenas, M. & Ryan, C. A. (1992) Structure, expression, and antisense inhibition of the systemin precursor gene. Science 255, 1570-1573.
Meyerowitz, E.M., Running, M.P., Sakai, H., and Williams, R.W. (1998) Multiple modes of cell division control in *Arabidopsis* flower development. Symp Soc Exp Biol 51, 19-26.
Murashige, T. and Skoog, F. (1960) A revised medium for rapid growth and bioassay with tobacco tissue cultures. Physiol. Plant. 15, 473-479.
Murry, M.G. and Thompson, W.F. (1980) Rapid isolation of high molecular weight plant DNA. Nucleic Acids Res. 8, 4321-4325.
Niehrs, C. and Huttner, W.B. (1990) Purification and characterization of tyrosylprotein sulfotransferase. EMBL J. 9, 35-42.
Pearce, G., Strydom, D., Johnson, S. & Ryan, C. A. (1991) A polypeptide from tomato leaves induces wound-inducible proteinase inhibitor proteins. Science 253, 895-898.
Reiter, R.S., Williams, J.G., Feldmann, K.A., Rafalski, J.A., Tingey, S.V. and Scolinik, P.A. (1992) Global and local genome mapping in *Arabidopsis thaliana* by using recombinant inbred lines and random amplified polymorphic DNAs. Proc Natl Acad Sci USA 89, 1477-1481.
The *Arabidopsis* Genome Initiative. (2000) Analysis of the genome sequence of the flowering plant *Arabidopsis* thaliana. Nature 408, 796-815.
Thompson, J.P., Higgins, D.G., and Gibson, T.J. (1994) CLUSTAL W: improving the sensitivity of progressive multiple sequence alignment through sequence weighting, position-specific gap penalties and weight matrix choice. Nucleic Acids Res. 22, 4673-4680.
Trotochaud, A.E., Jeong, S., and Clark, S.E. (2000) CLAVATA3, a multimeric ligand for the CLAVATA1 receptor-kinase. Science 289, 613-617.
Valvekens, D., Van Montagu, M., and Van Lijsebttens, M. (1988) *Agrobacterium tumefaciens*-mediated transformation of *Arabidopsis thaliana* root explants by using kanamycin selection. Proc. Natl. Acad. Sci. USA 85, 5536-5540.
Van Haute, E., Joos, H., Maes, S., Warren, G., Van Montagu, M., and Schell, J. (1983) Intergeneric transfer and exchange recombination of restriction fragments cloned in pBR322: a novel strategy for reversed genetics of the Ti plasmids of *Agrobacterium tumefaciens.* EMBO J. 2, 411-418.
Wendel, J.F. (2000) Genome evolution in polyploids. Plant Mol. Biol. 42, 225-249.
Yamakawa, S., Matsubayashi, Y., Sakagami, Y., Kamada, H., and Satoh, S. (1998a) Promotion by a peptidyl plant growth factor, phytosulfokine-α, of chlorophyll formation in etiolated cotyledons of cucumber. Biosci. Biotechnol. Biochem. 62, 2441-2443.
Yamakawa, S., Sakurai, C., Matsubayashi, Y., Sakagami, Y., Kamada, H., and Satoh, S. (1998b) The promotive effects of a peptidyl plant growth factor, phytosulfokine, on the formation of adventitious roots and expression of a gene for a root-specific cystatin in cucumber hypocotyls. J. Plant Res. 111, 453-458.
Yamakawa, S., Matsubayashi, Y., Sakagami, Y., Kamada, H., and Satoh, S. (1999) Promotive effects of the peptidyl plant growth factor, phytosulfokine-α, on the growth and chlorophyll content of *Arabidopsis* seedlings under high night-time temperature conditions. Biosci. Biotechnol. Biochem. 63, 2240-2243.
Yang, G., Shen, S., Kobayashi, T., Matsubayashi, Y., Sakagami, Y., and Kamada, H. (1999a) Stimulatory effects of a novel peptidyl plant growth factor, phytosulfokine-α, on adventitious bud formation in *Antirrhinum majus.* Plant Biotechnol. 16, 231-234.
Yang, H., Matsubayashi, Y., Nakamura, K. and Sakagami, Y. (1999b) *Oryza sativa PSK* encodes a precursor of phytosulfokine-α, a sulfated peptide growth factor found in plants. Proc. Natl. Acad. Sci. USA 96, 13560-13565.
Yang, H., Matsubayashi, Y., Hanai, H., and Sakagami, Y. (2000a) Phytosulfokine-α, a peptide growth factor found in higher plants: its structure, functions, precursor and receptors. Plant Cell Physiol. 41, 825-830.
Yang, H., Matsubayashi, Y., Hanai, H., Nakamura, K. and Sakagami, Y. (2000b) Molecular cloning and characterization of *OsPSK*, a gene encoding a precursor for phytosulfokine-α, required for rice cell proliferation. Plant Mol. Biol. 44, 635-647.

## Claims

1. A precursor polypeptide of phytosulfokine derived from *Arabidopsis thaliana*, said precursor polypeptide being following (a) or (b):
(a) a polypeptide consisting of an amino acid sequence defined as amino acid numbers from 1 to 87 in SEQ ID NO: 1 in the sequence list,
(b) a polypeptide consisting of an amino acid sequence having more than 70% of sequence homology with said polypeptide (a), said polypeptide (b) being processed and sulfated on the tyrosine residue of said phytosulfokine in a plant cell to secrete said phytosulfokine with its tyrosine residue sulfated, thereby capable of promoting proliferation of said plant cell.

2. A gene encoding said precursor polypeptide of phytosulfokine according to claim 1.

3. A gene encoding said precursor polypeptide of phytosulfokine according to claim 1, said gene consisting of a base sequence defined as base numbers from 40 to 303 in SEQ ID NO: 2 in the sequence list.

4. A gene encoding a precursor polypeptide of phytosulfokine derived from *Arabidopsis thaliana,* said gene being following (c), (d) or (e):
(c) a gene consisting of a base sequence defined as base numbers from 1 to 545 in SEQ ID NO: 2 in the sequence list,
(d) a gene consisting of a base sequence that hybridizes with the base sequence (c) under a stringent condition,
(e) a gene consisting of a base sequence having more than 70% of sequence homology with said gene (c), said gene (e) encoding a polypeptide being processed and sulfated on the tyrosine residue of said phytosulfokine in a plant cell to secrete said phytosulfokine with its tyrosine residue sulfated, thereby capable of promoting proliferation of said plant cell.

5. A method to promote proliferation of a plant cell, the method comprising incorporation of said gene according to either one of claim 2 to claim 4 into said plant cell.

6. A transgenic plant cell comprising said gene according to either one of claim 2 to claim 4 incorporated therein to promote proliferation of said plant cell.

7. A precursor polypeptide of phytosulfokine derived from *Arabidopsis* *thaliana,* said precursor polypeptide being following (f) or (g) :
(f) a polypeptide consisting of an amino acid sequence defined as amino acid numbers from 1 to 79 in SEQ ID NO: 3 in the sequence list,
(g) a polypeptide consisting of an amino acid sequence having more than 70% of sequence homology with said polypeptide (f), said polypeptide (g) being processed and sulfated on the tyrosine residue of said phytosulfokine in a plant cell to secrete said phytosulfokine with its tyrosine residue sulfated, thereby capable of promoting proliferation of said plant cell.

8. A gene encoding said precursor polypeptide of phytosulfokine according to claim 7.

9. A gene encoding said precursor polypeptide of phytosulfokine according to claim 7, said gene consisting of a base sequence defined as base numbers from 43 to 282 in SEQ ID NO: 4 in the sequence list.

10. A gene encoding a precursor polypeptide of phytosulfokine derived from *Arabidopsis thaliana*, said gene being following (h) , (i) or (j):
(h) a gene consisting of a base sequence defined as base numbers from 1 to 479 in SEQ ID NO: 4 in the sequence list,
(i) a gene consisting of a base sequence that hybridizes with the base sequence
(h) under a stringent condition,
(j) a gene consisting of a base sequence having more than 70% of sequence homology with said gene (h), said gene (j) encoding a polypeptide being processed and sulfated on the tyrosine residue of said phytosulfokine in a plant cell to secrete said phytosulfokine with its tyrosine residue sulfated, thereby capable of promoting proliferation of said plant cell.

11. A method to promote proliferation of a plant cell, the method comprising incorporation of said gene according to either one of claim 8 to claim 10 into said plant cell.

12. A transgenic plant cell comprising said gene according to either one of claim 8 to claim 10 incorporated therein to promote proliferation of said plant cell.


Sequence listing
<110> President of Nagoya University
<120> A PRECURSOR POLYPEPTIDE OF PHYTOSULFOKINE DERIVED
FROM ARABIDOPSIS THALIANA AND A GENE ENCODING SAID
POLYPEPTIDE
<160> 4
<210> 1
<211> 87
<212> Amino Acid
<213> Seeds of Arabidopsis thaliana
<400>
MANVSALLTI ALLLCSTLMC TARPEPAISI SITTAADPCN MEKKIEGKLD DMHMVEENCG 60
ADDEDCLMRR TLVAHTDYIY TQKKKHP 87
<210> 2
<211> 545
<212> Nucleic Acid
<213> Seeds of Arabidopsis thaliana

<400>

TTTAAGCATC TCTCTCCCTT CAAAGAAAAT ATCACAAAAA TGGCAAACGT CTCCGCTTTG 60

CTCACCATAG CTCTTCTCCT TTGCTCCACG CTAATGTGCA CTGCCCGCCC CGAACCGGCC 120

ATCTCCATCT CTATCACGAC TGCTGCCGAT CCATGTAACA TGGAGAAGAA GATAGAAGGA 180

AAATTAGATG ACATGCATAT GGTAGACGAA AACTGTGGTG CAGACGACGA AGATTGCTTA 240

ATGAGGAGGA CTTTGGTCGC TCATACTGAT TACATCTATA CCCAGAAGAA GAAGCATCCT 300

TGATTTTCAC TTACTCATTT CTAACAAACT TTTGCTCAAG ATTATGTAAT TTATGTCATC 360

CTTAATTAGT ATTTGTCACA TACAAGATCA AGATAGTTAT GATTGTGATT ATTTATATGT 420

ATTTTGGATG ATATATTGTA GAACTAGAAG TTTGACAATA AAAAGTTTAT AGTGTCGTTG 480

TATGTTTAAG AAGTTTGACA ATAAAAACTT TATAGTGTTG TTGCAAAAAA AAAAAAAAAA 540

AAAAA 545

<210> 3

<211> 79

<212> Amino Acid

<213> Seeds of Arabidopsis thaliana

<400>

MGKFTTIFIM ALLLCSTLTY AARLTPTTTT ALSRENSVKE IEGDKVEEES CNGIGEEECL 60

IRRSLLLHTD YIYTQNHKP 79

<210> 4

<211> 479

<212> Nucleic Acid

<213> Seeds of Arabidopsis thaliana

<400>

CTTCTCAGGC TCCCATTATC TTTCTCTATT TTGCAAATCA GTATGGGTAA GTTCACAACC 60

ATTTTCATCA TGGCTCTCCT TCTTTGCTCT ACGCTAACCT ACGCAGCAAG GCTGACTCCG 120

ACGACAACCA CCGCTTTGTC CAGAGAAAAC TCCGTCAAGG AAATTGAAGG AGACAAGGTT 80

GAAGAAGAAA GCTGCAACGG AATTGGAGAA GAAGAATGTT TGATAAGACG AAGCCTTCTT 240

CTTCACACCG ATTACATTTA TACTCAGAAT CACAAGCCCT AAGTTTCTGT ATTAGAGCAA 300

TTAATTAACT AATTACATTA TCAAACCTAT CACTGTAGTA CTTTCTGTTT TCTGTTCGTC 360

TTTTTTGTTT TGTTTTGTTT ATGTTATCGC TATTTTAAAG TTTCATTGTT ATTTTGAAAG 420

TTCTTACACC ATCATTATTG ATAGTCTCTT AAAACAAAAA AAAAAAAAAA AAAAAAAAA 479

### Annex to the application documents - subsequently filed sequences listing

SEQUENCE LISTING

<110> NAGOYA UNIVERSITY

<120> A precursor polypeptide of phytosulfokine derived from
      Arabidopsis thaliana and a gene encoding said
      polypeptide

<130> EP-88068

<140> 02003238.9
<141> 2002-02-20

<160> 4

<170> PatentIn Ver. 2.1

<210> 1
<211> 87
<212> PRT
<213> Seeds of Arabidopsis thaliana

<400> 1
Met Ala Asn Val Ser Ala Leu Leu Thr Ile Ala Leu Leu Leu Cys Ser
  1               5                  10                  15

Thr Leu Met Cys Thr Ala Arg Pro Glu Pro Ala Ile Ser Ile Ser Ile
             20                  25                  30

Thr Thr Ala Ala Asp Pro Cys Asn Met Glu Lys Lys Ile Glu Gly Lys
         35                  40                  45

Leu Asp Asp Met His Met Val Glu Glu Asn Cys Gly Ala Asp Asp Glu
     50                  55                  60

Asp Cys Leu Met Arg Arg Thr Leu Val Ala His Thr Asp Tyr Ile Tyr
 65                  70                  75                  80

Thr Gln Lys Lys Lys His Pro
                 85

<210> 2
<211> 545
<212> DNA
<213> Seeds of Arabidopsis thaliana

<400> 2
tttaagcatc tctctccctt caaagaaaat atcacaaaaa tggcaaacgt ctccgctttg 60
ctcaccatag ctcttctcct ttgctccacg ctaatgtgca ctgcccgccc cgaaccggcc 120
atctccatct ctatcacgac tgctgccgat ccatgtaaca tggagaagaa gatagaagga 180
aaattagatg acatgcatat ggtagacgaa aactgtggtg cagacgacga agattgctta 240
atgaggagga ctttggtcgc tcatactgat tacatctata cccagaagaa gaagcatcct 300
tgattttcac ttactcattt ctaacaaact tttgctcaag attatgtaat ttatgtcatc 360
cttaattagt atttgtcaca tacaagatca agatagttat gattgtgatt atttatatgt 420
attttggatg atatattgta gaactagaag tttgacaata aaaagtttat agtgtcgttg 480
tatgtttaag aagtttgaca ataaaaactt tatagtgttg ttgcaaaaaa aaaaaaaaaa 540
aaaaa                                                             545

<210> 3
<211> 79
<212> PRT
<213> Seeds of Arabidopsis thaliana

<400> 3

Met Gly Lys Phe Thr Thr Ile Phe Ile Met Ala Leu Leu Leu Cys Ser
1               5                   10                  15

Thr Leu Thr Tyr Ala Ala Arg Leu Thr Pro Thr Thr Thr Thr Ala Leu
            20                  25                  30

Ser Arg Glu Asn Ser Val Lys Glu Ile Glu Gly Asp Lys Val Glu Glu
        35                  40                  45

Glu Ser Cys Asn Gly Ile Gly Glu Glu Glu Cys Leu Ile Arg Arg Ser
    50                  55                  60

Leu Leu Leu His Thr Asp Tyr Ile Tyr Thr Gln Asn His Lys Pro
65                  70                  75

<210> 4
<211> 479
<212> DNA
<213> Seeds of Arabidopsis thaliana

<400> 4
cttctcaggc tcccattatc tttctctatt ttgcaaatca gtatgggtaa gttcacaacc 60
attttcatca tggctctcct tctttgctct acgctaacct acgcagcaag gctgactccg 120
acgacaacca ccgctttgtc cagagaaaac tccgtcaagg aaattgaagg agacaaggtt 180
gaagaagaaa gctgcaacgg aattggagaa gaagaatgtt tgataagacg aagccttctt 240
cttcacaccg attacattta tactcagaat cacaagccct aagtttctgt attagagcaa 300
ttaattaact aattacatta tcaaacctat cactgtagta ctttctgttt tctgttcgtc 360
ttttttgttt tgttttgttt atgttatcgc tattttaaag tttcattgtt attttgaaag 420
ttcttacacc atcattattg atagtctctt aaaacaaaaa aaaaaaaaaa aaaaaaaaa 479